# EUROPEAN PATENT SPECIFICATION

(11) **EP 1 711 507 B1**
(45) Date of publication and mention of the grant of the patent: **03.12.2008**
(21) Application number: 05700509.2
(22) Date of filing: 04.02.2005
(51) Int. Cl.: C07F 9/58, A61K 31/663, A61P 19/00

(54) **CRYSTALLINE FORM OF RISEDRONATE MONOSODIUM**
KRISTALLINE FORM VON RISEDRONAT-MONONATRIUM
FORME CRISTALLINE DU RISEDRONATE MONOSODIQUE

(30) Priority: 05.02.2004 CZ 20040199
(43) Date of publication of application: 18.10.2006
(73) Proprietor: Zentiva, a.s., 102 37 Praha 10 (CZ)
(72) Inventor: RICHTER, Jindrich, 530 12 Pardubice (CZ); JIRMAN, Josef, 100 00 Praha 10 (CZ)
(74) Representative: Jirotkova, Ivana
(86) International application number: PCT/CZ2005/000012
(87) International publication number: WO 2005/075487

(56) References cited:
- WO-A-03/086355

## Description

### Technical Field

The invention concerns a new crystalline form of the salt of 3-pyridyl-1-hydroxyethylidene-1,1-bisphosphonic acid (risedronic acid) 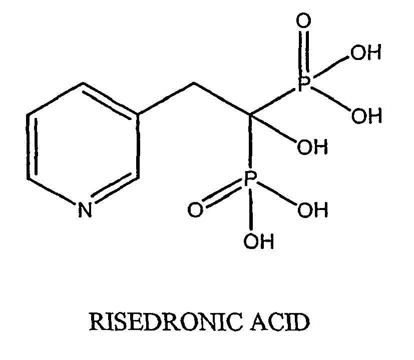 and a method of its preparation.

### Background Art

Geminal bisphosphates, such as for example salts of 3-pyridyl-1-hydroxyethylidene-1,1-bisphosphonic acid (RISEDRONATE) or 4-amino-1-hydroxybutylidene-1,1- bisphosphonic acid (ALENDRONATE) have already been used for some time to treat bone diseases and to adjust calcium metabolism.

Preparation of risedronic acid consists in the reaction of 3-pyridylacetic acid with phosphorous acid and phosphorus trichloride and subsequent hydrolysis of the resulting intermediates. A general method of this preparation of bisphosphonic acids was presented in JP 80-98193 (1980), JP 80-98105 (1980) of Nissan Chemical Industries and in the article of W. Ploger et al., Z. Anorg. Allg. Chem., 389, 119 (1972). Preparation of risedronate was presented in EP 186405 (1986) of Procter & Gamble.

Bisphosphonic acids are used in the form of various nontoxic and pharmaceutically acceptable esters, salts of alkali metals and alkaline-earth metals and their various hydrates. The form of the substance can have fundamental influence on its solubility and biological availability. The sodium and calcium salts are the preferred forms of risedronate.

3-pyridyl-1-hydroxyethylidene-1,1-bisphosphonic acid is used especially in the form of the monosodium salt (SODIUM RISEDRONATE). This salt, like a number of germinal bisphosphonic acids and their salts, is able to form hydrates. So far, anhydrous crystalline form of monosodium 3-pyridyl-1-hydroxyethylidene-1,1-bisphosphate, its monohydrate and pentahemihydrate have been described in the Procter & Gamble's application WO 0156983 A2. Of the two mentioned hydrates, only the pentahemihydrate form is thermodynamically stable. The monohydrate spontaneously transforms to the stabile pentahemihydrate.

The dosage form containing monohydrate, described in the application WO 01/56983, therefore, necessarily absorbs water from the environment and changes its composition. This can be a significant source of instability of this form.

In our earlier patent application No. WO 2004/037252 we have described higher hydrates of the monosodium, di-sodium and tri-sodium salts of 3-pyridyl-1-hydroxyethylidene-1,1-bisphosphonic acid. It has turned out that these salts do not take in any additional water from the air and it could be assumed that a formulation containing them would be more stable than that of a mixture of the mono- and pentahemihydrate of the monosodium salt described earlier.

New crystalline modifications have been described in the patent application WO 03/086355. The structure of the earlier-known hemipentahydrate has been designated therein as polymorph A. In the invention, other forms B through H are defined.

Methods of preparation of individual forms consist either in refluxing a suspension of risedronic acid in a solution of sodium hydroxide in a mixture water-organic solvent, or in heating the monosodium salt to 100 - 200 °C, or in exposing the salt to wet atmosphere for long periods of time.

According to this patent application, the H form is formed by the third mentioned method. However, it has now turned out that the H form transforms, at slightly elevated temperature, to another so-far-not-described form J. The J form is stable under normal conditions for storing pharmaceuticals.

### Disclosure of Invention

The invention consists in a new crystalline modification I of the sodium salt of 3-pyridyl-1-hydroxyethylidene-1,1- bisphosphonic acid. The J modification is characterized by X-ray diffraction and infrared spectra.

The X-ray diffraction pattern of this crystalline modification provides characteristic interplanar distances 4.35; 8.44; 13.42 and 15.73 Å. This crystalline modification further provides characteristic bands of FTIR spectrum 529; 887; 1064 and 1172 cm⁻¹.

The new crystalline form can be obtained from the H form by subjecting it to temperatures of 40 - 60 °C for 3 - 10 hours. A temperature about 50 °C, when the change takes place in a reasonable rate on one hand and selective product results on the other hand, turns out to be the most advantageous one. The J form has turned out to be stable if it is stored under normal conditions.

### Brief Description of Drawings

Figure 1 shows the X-ray diffraction pattern of the crystalline modification according to the invention.
Figure 2 shows infrared spectra of the crystalline modification according to the invention.
Figure 3 shows the X-ray diffraction pattern of crystalline modification H according to WO 03/086355.
Figure 4 shows the X-ray diffraction pattern of the same lot of Form H as in Figure 3, after approximately 5 months.
Figure 5 shows three X-ray diffraction patterns of crystalline modification J according to the invention; the middle spectra being measured 10 days later than the lower one and the upper spectra being measured 9 months after the lower one.

### Examples

The invention is further illustrated with the following example.

### Example 1

Monosodium risedronate of the H crystalline form was dried at 50 °C for 5 hours. The resulting product has stable structure in the atmosphere.

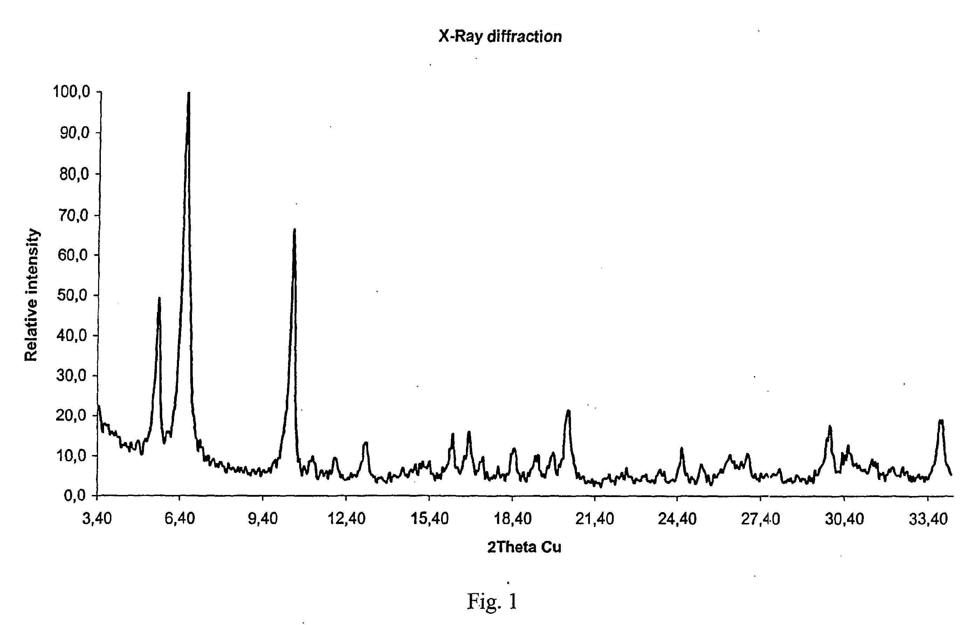

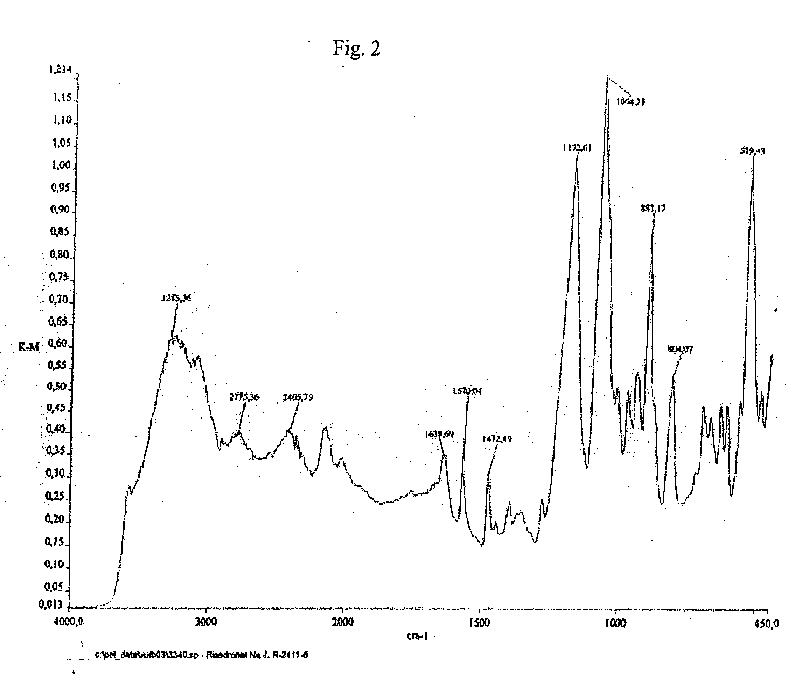

The crystalline form of the product was characterized with the X-ray diffraction pattern in Figure 1 and infrared spectra in Figure 2.

The recorded reflections in cobalt X-ray and copper X-ray radiation and calculated interplanar distances are presented in the following table.

| 2θ_{Co} | d | 2θ_{Cu} | Iᵣₑₗ |
|---|---|---|---|
| 6.52 | 15.73 | 5.61 | 47.30 |
| 7.65 | 13.42 | 6.58 | 100.00 |
| 12.17 | 8.44 | 10.46 | 66.10 |
| 15.24 | 6.75 | 13.10 | 14.05 |
| 18.85 | 5.47 | 16.20 | 14.76 |
| 19.56 | 5.27 | 16.81 | 14.17 |
| 23.75 | 4.35 | 20.40 | 22.11 |
| 34.85 | 2.99 | 29.86 | 17.59 |
| 39.54 | 2.65 | 33.83 | 19.41 |

A comparison of the crystalline form according to the invention, designated Form J, with crystalline form H according to WO 03/086355:

### Stability of sodium risedronate, crystalline form H:

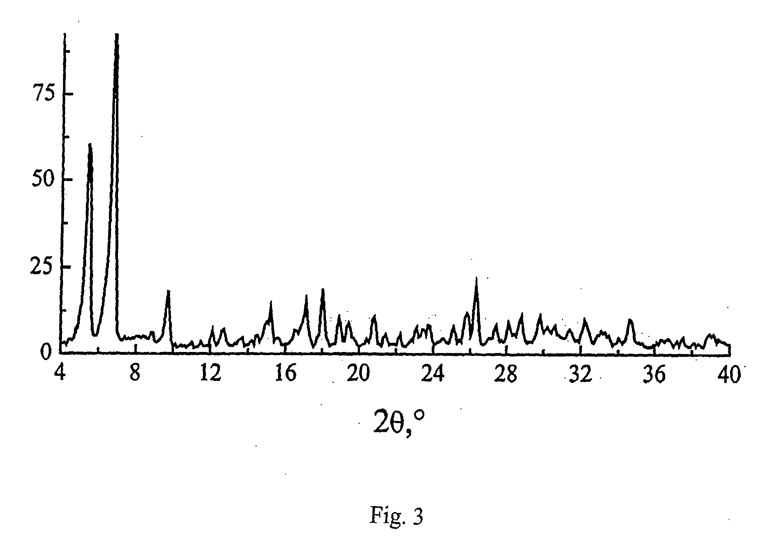

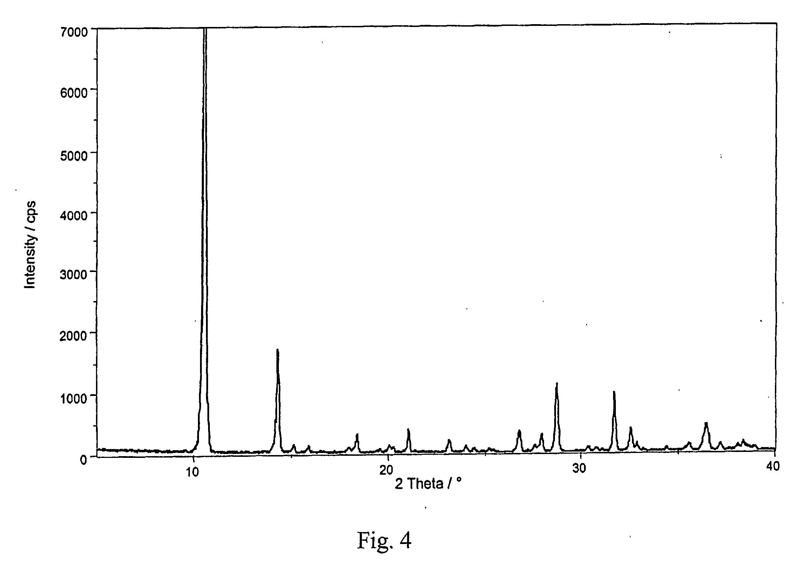

A sample of sodium risedronate, crystalline form H, in the amount of about 5 g, identified by means of X-ray diffraction (Fig. 3), was hermetically sealed in a polyethylene bag and left alone at room temperature (20 - 25 °C). After almost 5 months (the sample was charged on 27 June 2003 and the test was completed on 7 November 2003) it was possible to state that absolute change of the crystalline form has occurred (Fig. 4). Above all, the two reflections typical for Form H at lower angles (< 8°) have completely disappeared; further an important reflection at 2 theta values of about 10.5 ° has appeared. Accordingly, crystalline form could not be identified in the resulting product.

### Stability of sodium risedronate, crystalline form J:

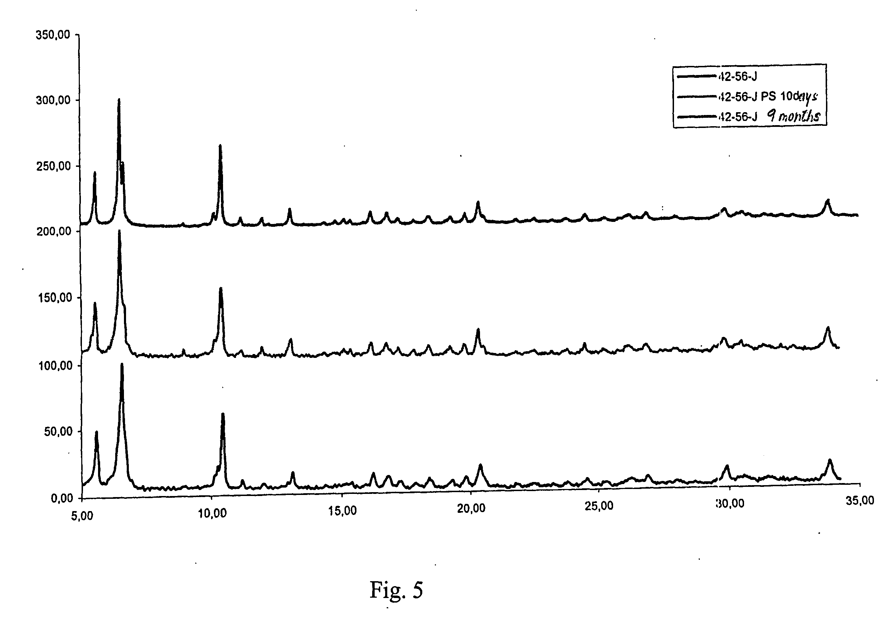

Form J, obtained according to Example 1, in the amount of about 5 g, identified by X-ray diffraction pattern (Fig. 5, lower spectra), was hermetically sealed in a glass container at room temperature, i.e. at about 20 - 25 °C. Samples were taken from the container twice, namely roughly after 10 days and after 9 months (Fig. 5). In all the three diffraction patterns in Fig. 5 the Form J can be reliably identified. Especially the characteristic reflections at lower 2 theta angles (< 10 °) are the determinant, most intensive formations in all the three measurements. In other, less intensive, reflections no significant differences have been found either. The measurements have demonstrated that Form J is more stable and, as contrasted to Form H, it can be used, from the point of view of polymorphic stability, for pharmaceutical compositions.

## Claims

1. Crystalline monosodium salt of 3-pyridyl-1-hydroxyethylidene-1,1-bisphosphonic acid having the X-ray pattern with characteristic interplanar distances 4.35; 8.44; 13.42 and 15.73 Å.

2. The crystalline salt according to claim 1, having the X-ray pattern with further characteristic interplanar distances 2.65; 2.99; 5.27; 5.47 and 6.75 Å.

3. The crystalline salt according to claim 1 or 2, having the FTIR record with characteristic bands 529; 887; 1064 and 1172 cm⁻¹.

4. The crystalline salt according to claim 3, having the FTIR record with further characteristic bands 804; 1472; 1570 and 1638 cm⁻¹.

5. A method of preparation of the crystalline salt according to any of the preceding claims **characterized in that** the crystalline monosodium salt of 3-pyridyl-1-hydroxyethylidene-1,1-bisphosphonic acid of the H crystalline form is heated at 40 - 60 °C for 3-10 hours.

6. The method according to the claim 5 **characterized in that** the salt is heated at 50 °C for 5 hours.

## Patentansprüche

1. Kristallines Mononatriumsalz der 3-Pyridyl-1-hydroxyethyliden-1,1-bisphosphonsäure mit dem RTG-Beugungsbild, enthaltend die charakteristischen Gitterabstände 4,35; 8,44; 13,42 und 15,73 Å.

2. Kristallines Salz nach Anspruch 1, mit dem RTG-Beugungsbild, enthaltend die weiteren charakteristischen Gitterabstände 2,65; 2,99; 5,27; 5,47 und 6,75 Å.

3. Kristallines Salz nach Anspruch 1 oder 2, mit dem FTIR-Spektrogramm, enthaltend die charakteristischen Bänder bei 529, 887, 1064 und 1172 cm⁻¹.

4. Kristallines Salz nach Anspruch 3, mit dem FTIR-Spektrogramm, enthaltend die weiteren charakteristischen Bänder bei 804, 1472, 1570 und 1638 cm⁻¹.

5. Verfahren zur Herstellung des kristallinen Salzes nach einem der vorhergehenden Ansprüche, **dadurch gekennzeichnet, dass** man das kristalline Mononatriumsalz der 3-Pyridyl-1-hydroxyethyliden-1,1-bisphosphonsäure in der H-Kristallform 3 - 10 Stunden bei 40 - 60 °C erhitzt.

6. Verfahren nach Anspruch 5, **dadurch gekennzeichnet, dass** man das Salz 5 Stunden bei 50 °C erhitzt.

## Revendications

1. Sel monosodium cristallin de l'acide 3-pyridyl-1-hydroxyéthylidène-1,1-bisphosphonique, ayant le diphractogramme radiographique avec des distances inter-réticulaires caractéristiques 4,35 ; 8,44 ; 13,42 et 15,73 Å.

2. Sel cristallin selon la revendication 1, ayant le diphractogramme radiographique avec d'autres distances inter-réticulaires caractéristiques 2,65 ; 2,99 ; 5,27 ; 5,47 et 6,75 Å.

3. Sel cristallin selon la revendication 1 ou 2, ayant le spectrogramme FTIR avec des bandes caractéristiques 529 ; 887 ; 1064 et 1172 cm⁻¹.

4. Sel cristallin selon la revendication 3, ayant le spectrogramme FTIR avec d'autres bandes caractéristiques 804 ; 1472 ; 1570 et 1638 cm⁻¹.

5. Procédé de préparation du sel cristallin selon l'une quelconque des revendications précédentes, **caractérisé en ce que** l'on chauffe le sel monosodium cristallin de l'acide 3-pyridyl-1-hydroxyéthylidène-1,1-bisphosphonique de forme cristalline H à 40 - 60 °C pendant 3-10 heures.

6. Procédé selon la revendication 5, **caractérisé en ce que** l'on chauffe le sel à 50 °C pendant 5 heures.
